(19) Europäisches Patentamt

European Patent Office

Office européen des brevets

(11) EP 0 751 970 B1

(12) EUROPEAN PATENT SPECIFICATION

(45) Date of publication and mention
of the grant of the patent:
27.05.1998 Bulletin 1998/22

(51) Int Cl.6: **C08G 59/12**, C07D 303/16,
C09D 163/00, C09D 5/03

(21) Application number: 95914307.4

(22) Date of filing: 23.03.1995

(86) International application number:
PCT/EP95/01124

(87) International publication number:
WO 95/25762 (28.09.1995 Gazette 1995/41)

(54) **OUTDOOR DURABLE POWDER COATING COMPOSITIONS**

WITTERUNGSBESTÄNDIGE PULVERBESCHICHTIGUNGSMASSE

COMPOSITIONS POUR REVETEMENTS EXTERIEURS DURABLES A BASE DE POUDRE

(84) Designated Contracting States:
BE CH DE ES FR GB IT LI NL SE

(30) Priority: 24.03.1994 EP 94200776

(43) Date of publication of application:
08.01.1997 Bulletin 1997/02

(73) Proprietor: SHELL INTERNATIONALE
RESEARCH MAATSCHAPPIJ B.V.
2596 HR Den Haag (NL)

(72) Inventors:
• FRERIKS, Jan
NL-1031 CM Amsterdam (NL)
• VAN GAALEN, Ronald, Petrus, Clemens
NL-1031 CM Amsterdam (NL)
• KOOYMANS, Petrus, Gerardus
NL-1031 CM Amsterdam (NL)
• RAUDENBUSCH, Werner, Theodor
NL-1031 CM Amsterdam (NL)

(56) References cited:
WO-A-94/04589          DE-B- 1 030 824
US-A- 3 576 903

• CHEMICAL ABSTRACTS, vol. 86, no. 20, 16 May
1977, Columbus, Ohio, US; abstract no. 140711h,
'Glycidyl esters' page 17 ; & JP,A,7 703 700 (UBE
INDUSTRIES LTD.) 12 January 1977

Remarks:
The file contains technical information submitted
after the application was filed and not included in this
specification

**Description**

The present invention relates to a polyglycidylester of an acid functional polyester, to a process for the preparation thereof, to a powder coating composition comprising such a polyglycidylester, to a process for the preparation of such a powder coating composition, to the use of such a powder coating composition for coating a substrate, and to substrates comprising such a powder coating composition in the cured state.

Powder coating compositions have been known in the art for some time and have recently gained interest for obvious environmental reasons due to the absence of solvents in these compositions.

Probably the best known powder coating compositions are those based on solid reaction products of 2,2-bis-(4-hydroxyphenyl)propane with an epihalohydrin. These compositions however have as serious drawback that their outdoor durability in the cured state is poor and as a result thereof these compositions have restricted end-use. Powder coating compositions based on conventional amorphous polyesters and suitable curing agents do show improved outdoor durability in the cured state.

Triglycidylisocyanurate (TGIC) is possibly the most commonly applied curing agent for such outdoor durable coating compositions. TGIC however is currently viewed with suspicion for health and safety reasons since it is relatively toxic (Rat Oral $LD_{40}$ of 0.4 g/kg) and is mutagenic according to the Ames Mutagenicity Test.

In view of the above there is a need for resin systems, particularly suitable for use in powder coating compositions, which provide good outdoor durability in the cured state and which, as compared to the currently available polyester/TGIC systems, are relatively non toxic.

Therefore the present invention relates to a semi-crystalline polyglycidylester obtainable by glycidation of the acid groups of a semi-crystalline acid functional polyester the latter being a reaction product of at least compound(s) A and compound(s) B, wherein compound A is a straight chain aliphatic alpha,omega-dicarboxylic acid having at least 6 carbon atoms, and wherein compound B is a straight chain aliphatic alpha,omega-primary diol having at least 4 carbon atoms and optionally a compound C, having at least two functional groups reactive with hydroxyl and/or carboxyl groups and not falling within the definition, for A and B, in an amount less than 25 %wt based on the total weight of compounds A, B and C, said reaction product having an acid content of from $\geq$ 1.6 meq/g to $\leq$ 4.0 meq/g, a number average molecular weight of at least 500, a melting temperature (Tm) by Differential Scanning Calorimetry of $\geq$ 25 °C, an average acid functionality of 2 or more and essentially no free reactive groups other than carboxyl groups.

The term "essentially no free reactive groups other than carboxyl groups" implies that at most 5 mole % of the "reactive groups other than carboxyl groups" as initially present in the reaction mixture, may be present as unreacted groups in the semi-crystalline acid functional polyester.

In view of the polymeric nature of the semi-crystalline polyglycidylesters of the invention, they are expected to be less toxic than TGIC.

Powder coating compositions based on an amorphous acid functional polyester and a semi-crystalline polyglycidylester according to the present invention show good flow out and a high level of outdoor durability in the cured state, and good mechanical properties in the cured state.

The polyglycidyl esters of the present invention and their precursor acid functional polyesters are both semi-crystalline. The concept of semi-crystallinity is well known in polymer chemistry (see for example R.J. Young: "Introduction to polymers" Chatham and Hall; London, New York 1983; pages 168 to 185). Semi-crystalline polymeric compositions are characterised by at least two phases, an amorphous and a crystalline phase. Further they are opaque at room temperature if they have a Tm value higher than room temperature. Semi-crystallinity can be demonstrated by Differential Scanning Calorimetry (DSC). When subjecting a semi-crystalline composition to a DSC experiment a melting effect and usually a glass-rubber transition temperature should be observed.

Generally the conversion of conventional amorphous acid functional polyesters into their corresponding glycidyl derivatives is accompanied by a significant decrease in the melting range of the glycidylester relative to the precursor polyester. This is mainly due to the formation of considerable amounts of lower molecular weight breakdown products due to hydrolysis of polyester linkages under strong alkaline conditions prevailing during glycidation. The observance of a decrease of the melting range of from 50 to 100 °C, when comparing the glycidated amorphous acid functional polyester with its non glycidated amorphous acid functional polyester counterpart, is not unusual and therefore these polyglycidylesters often melt at low temperatures which renders them not suitable for use in powder coating compositions.

Having in mind the above phenomenon it was surprising to find that the semi-crystalline polyglycidylesters of the present invention as compared to their precursor semi-crystalline acid functional polyesters only show a minimal change in the Tm value which indicates that low molecular weight products resulting from polyester hydrolysis have not or hardly been formed, a finding which can be demonstrated by gel permeation chromatography (GPC) analysis.

As indicated above the semi-crystalline acid functional polyester compound wherefrom the semi-crystalline polyglycidylester of the present invention can be obtained is prepared from "at least compound(s) A and compound(s) B". This definition thus encompasses:

- one compound A and one compound B,
- one compound A and two or more different compounds B,
- two or more different compounds A and one compound B,
- two or more different compounds A and two or more different compounds B, and
- any one of the above combinations of compounds A and B and in addition a suitable amount of one or more suitable compound(s) C, which have at least two functional groups reactive with hydroxyl- and or carboxyl groups, and which do not fall within the definitions of compounds A and B given above in an amount less than 25 %wt based on the total weight of compounds A, B and C.

Suitable compounds A are for example adipic -, pimelic -, suberic -, azelaic -, sebacic- and 1,10-decanedicarboxylic acid.

Suitable compounds B are for example 1,4-butanediol, 1,5-pentanediol, 1,6-hexanediol, 1,7-heptanediol, 1,8-octanediol, 1,9-nonane diol, 1,10-decanediol and 1,12-dodecanediol.

Those compounds A and B that have an even number of carbon atoms are particularly preferred, in view of their higher melting points. 1,10-decanedicarboxylic acid and 1,6-hexanediol are particularly preferred compounds A and B respectively.

When the semi-crystalline acid functional polyester is prepared using a specified amount of a specific compound C, the glycidylester derivative thereof will have physical properties which reflect the nature of this compound C. Therefore in practice the decision whether or not to use a certain amount of one or more specific compound(s) C for the preparation of the semi-crystalline acid functional polyester will depend on the wish to obtain a polyglycidylester having one or more desirable specific properties.

Suitable compounds C may for example be selected from the following groups of compounds:

1) Aliphatic or alicyclic diprimary amines, such as for example 1,6-hexanediamine, bis(4-aminocyclohexyl)methane or isophoronediamine. These compounds introduce amide linkages that form hydrogen bridges which increase the Tm value and increase the viscosity of the semi-crystalline acid functional polyester.

2) Aromatic or cycloaliphatic polycarboxylic acids or the corresponding anhydrides, having at least two carboxyl groups per molecule, such as terephthalic acid, isophthalic acid, 1,4-cyclohexanedicarboxylic acid, hexahydrophthalic anhydride and trimellitic anhydride. The cyclic structures may have the effect of raising the Tg and the viscosity of the semi-crystalline acid functional polyester, whilst compounds having more than two carboxyl groups in addition introduce a certain extent of branching whereby the viscosity of the semi-crystalline acid functional polyester may be further increased.

3) Polyols having at least three hydroxyl groups such as 1,1,1-trimethylolpropane, 1,1,1-trimethylolethane, ditrimethylolpropane, 1,2,6,hexanetriol, glycerol and pentaerythritol. These polyols introduce branching and thus increase the viscosity of the semi-crystalline acid functional polyester.

4) Compounds having simultaneously carboxyl- and hydroxyl groups and having a functionality of three or more, such as dimethylolpropionic acid or 2,2-dimethylol-1-butanoic acid. These compounds increase the acid functionality of the semi-crystalline acid functional polyester.

When the semi-crystalline acid functional polyester is prepared from one or more compound(s) A and one or more compound(s) B, approximately n + 1 moles of the total amount of compound(s) A is reacted with approximately n moles of the total amount of compound(s) B, wherein n should be chosen such that a semi-crystalline acid functional polyester having the claimed characteristics is obtained, which is a matter of routine for the skilled person.

Similarly, when in addition to compounds A and B one or more compound(s) C is being used for the preparation of the semi-crystalline acid functional polyester, the skilled person should chose the relative amounts of compounds A, B, and C such that a semi-crystalline acid functional polyester is obtained which has the characteristics claimed. This can be done as a matter of routine by the skilled person and the amount of compound C used should be less than 25% by weight based on the total weight of the compounds A, B, and C used.

The preferred number average molecular weight ($\overline{M}n$) of the semi-crystalline acid functional polyester is of from 500 to 4000 more preferably of from 1500 to 2500.

The preferred weight average molecular weight ($\overline{M}w$) of the semi-crystalline acid functional polyester is of from 3000 to 100,000 more preferably from 3000 to 60,000.

The preferred Tm value of the semi-crystalline acid functional polyester, lies in the range of from 40 to 70 °C, as determined by Differential Scanning Calorimetry (DSC).

The preferred acid functionality of the semi-crystalline acid functional polyester is of from 2.0 to 4.0, more preferably of from 2.0 to 3.5 carboxyl groups per molecule.

The preferred acid content of the semi-crystalline acid functional polyester is of from 1.6 to 3.0 meq/g, more preferably of from 2.0 to 3.0 meq/g

It was found that powder coating compositions according to the present invention comprising semi-crystalline polyglycidylesters obtained from semi-crystalline acid functional polyesters having one or more characteristics falling within the above preferred ranges provide the best overall properties.

In principle any conventional esterification and glycidation method can be used for the preparation of the semi-crystalline acid functional polyester and its glycidated derivative. Esterification is typically carried out via azeotropic condensation. In particular the condensation is carried out by charging all reactants and a suitable solvent such as toluene, xylene or methylisobutylketone to the reactor whereafter the temperature is increased from room temperature to from approximately 200 to 220 °C during a period of from 3 to 8 hours thus allowing the reaction to initiate and to proceed under continuous azeotropic removal of water. Generally the azeotropic removal of water is being continued until essentially all the hydroxyl groups as initially present in the reaction mixture have reacted. Generally this process is carried out in the absence of any catalyst however if necessary a suitable esterification catalyst may. be applied such as for example dibutyltinoxide, paratoluenesulfonic acid, tinoctoate, zincoctoate and lithiumricinoleate.

The glycidation of the semi-crystalline acid functional polyester is usually carried out by reaction of the acid functional polyester with an epihalohydrin, preferably epichlorohydrin, using from 5 to 40 moles of epihalohydrin per carboxyl group present in the reaction mixture, and in the presence of a suitable catalyst such as a tertiary amine or or - phosphine or a quaternary ammonium halide, followed by a dehydrohalogenation step in the presence of an alkaline reagent such as for example sodium hydroxide, potassium hydroxide, calcium hydroxide, sodium carbonate or potassium carbonate.

The present invention further provides a thermosetting powder coating composition comprising a semi-crystalline polyglycidylester of the present invention and at least one suitable organic compound having groups reactive with epoxy groups and optionally a catalyst.

Examples of such organic compounds are for example: solid polyacids such as sebacic acid, 1,10-decanedicarboxylic acid; acid anhydrides such as polyazeleic polyanhydride and trimellitic anhdyride; amorphous acid functional polyesters such as commercially available URALAC P 5500 and URALAC P 5400 (ex DSM), Crylcoat 2988 (ex UCB Chemicals), acid functional acrylic resins such as SCX 815, SCX 819 and SCX 839 (ex SC Johnson Polymer), a reaction product of one mole of trimethylolpropane and 3 moles of hexahydrophthalic anhydride and the reaction product of one mole of hexamethoxymethylmelamine and 3 moles of hydroxypivalic acid, and a linear tertiary aliphatic carboxyl functional polyester resin as disclosed in European patent application No. 93202081.1; cyanuric acid, and solid curing agents such as dicyandiamine and $BF_3$-amine complexes.

The powder coating compositions of the present invention may further comprise other additives that are typically used in powder coating compositions such as pigments, flow control agents, anti popping agents, tribostatic charge enhancing additives and stabilisers such as a UV light absorbing compound.

The ingredients of the powder coating compositions according to this invention can be blended by the processes known for producing powder coatings. Usually the ingredients are dry blended at ambient temperature followed by passing the premix through an extruder at a temperature sufficiently high to soften the semi-crystalline polyglycidylester and organic compound(s) but lower than the cure temperature, for example at a temperature of from 80 to 120 °C. Thereafter the extrudate is cooled and grinded to obtain a powder of the desired particle size usually between 10 and 250 microns.

The powder coating composition of the present invention can be applied to metal such as steel or to aluminum, glass, plastic or fibre reinforced substrates.

Application of the powder can be electrostatic (corona discharge or triboelectric) spraying or by the use of fluidized bed, electrostatic spraying being preferred. The coating is then heat cured at a temperature in the range of 150 °C to 225 °C most preferably at a temperature of from 160 to 200 °C.

The invention is further illustrated by the following examples.

In the examples the following test methods were used.

- The number- and weight average molecular weights were determined by gel permeation chromatograhpy (GPC) using polystyrene calibration.
- The Tm and Tg values (onset values) were determined on a Perkin Elmer DSC 7 instrument, heating mode using a heating rate of 20 °C/min.
- The melt viscosity was determined using the ICI cone and plate viscosimeter.
- The melting range was determined with a Kofler hot bench.

EXAMPLES

Example 1

a) Preparation of linear semi-crystalline acid functional polyester 1

4.0 moles of 1,10-Decanedicarboxylic acid (DDA), 3.0 moles of 1,6-hexanediol (HD) and xylene (5% on intake) were charged into a glass reaction flask equipped with anchor stirrer, thermometer, Dean-Stark water trap and nitogen inlet. The esterification reaction started at 170 °C and the reaction water formed was removed by azeotropic distillation. The temperature of the reaction mixture was raised to, and held at 220 °C until essentially all the hydroxyl functions had reacted. The mixture was cooled to about 180 °C and a vacuum (200 mmbar) was applied to remove the azeotropic solvent (xylene). Finally, vacuum was removed, the polyester was cooled to about 160 °C, discharged into an aluminium tray and allowed to cool to room temperature.

The product obtained was a white solid with the following properties:

| Molecular weight: | |
|---|---|
| $\overline{M}n$ | 1954 |
| $\overline{M}w$ | 3823 |
| Acid content (meq/g) | 1.72 |
| Acid functionality (theoretical) | 2.0 |
| Melting range (°C) | 65-67 |
| Tg* | - |
| Tm (°C) | 67 |
| Viscosity at 175 °C (mPa.s) | < 100 |

* Not determinable due to high degree crystallinity

b) Preparation of semi-crystalline polyglycidylester I

1 carboxyl equivalent polyester (1) (581 g), 25 moles epichlorohydrin (ECH) and tetramethylammonium chloride (1.1 g) were heated while stirring to 100 °C during 2 hours. Thereafter, a vacuum of about 200 mbar was applied, so that the reaction mixture boiled under reflux at 70 °C. While keeping the mixture at 70 °C, a 50% w/w solution of potassium hydroxide (1.05 moles solid KOH) was added over a period of one hour. During the addition, solid KCl was formed and water was removed azeotropically with the refluxing epichlorohydrin, the latter being fed back into the reactor after separating the water.

After the addition, ECH and other volatiles were removed under vacuum (100-200 mbar) while increasing the temperature to 100 °C.

Methylisobutylketone (3 litres) was added and the solids (mostly KCl) were filtered . The organic phase was successively washed with 10% w/w aqueous of $NaH_2PO_4$ and water.

After stripping the solvent under vacuum, the resulting white solid semi-crystalline product had the following properties:

| Molecular weight: | |
|---|---|
| $\overline{M}n$ | 1905 |
| $\overline{M}w$ | 3836 |
| Epoxy group content (found) (meq/g) | 1.51 |
| Epoxy group content (theoretical) (meq/g) | 1.57 |
| Melting range (°C) | 61-65 |
| Tg (°C) | -47 |
| Tm (°C) | 64 |
| Viscosity at 100 °C (mPa.s) | < 100 |

Example 2

a) Preparation of linear semi-crystalline acid functional polyester 2

Example 1a) was repeated using 4 moles adipic acid instead of 4 moles DDA.
The product obtained was a white solid with the following properties:

| Molecular weight (GPC): | |
|---|---|
| $\overline{M}n$ | 1502 |
| $\overline{M}w$ | 2770 |
| Acid content (meq/g) | 2.41 |
| Acid functionality (theoretical) | 2.0 |
| Tm (°C) | 50 °C |

b) Preparation of semi-crystalline polyglycidylester II

Preparation as in example 1b), using the linear semi-crystalline acid functional polyester 2 instead of 1.
The resulting white semi-crystalline product has the following properties:

| Molecular weight: | |
|---|---|
| $\overline{M}n$ | 1394 |
| $\overline{M}w$ | 2770 |
| Epoxy group content (found) (meq/g) | 2.03 |
| Epoxy group content (theoretical) (meq/g) | 2.12 |
| Tm (°C) | 45 °C |

Example 3

a) Preparation of linear semi-crystalline acid functional polyester 3 using a diamine as compound C

Example 1a) was repeated using a mixture of 2.4 moles HD and 0.27 moles 1,6 hexanediamine instead of 3 moles HD.
The product obtained was a white solid with the following properties:

| Molecular weight: | |
|---|---|
| $\overline{M}n$ | 1559 |
| $\overline{M}w$ | 2890 |
| Acid content (meq/g) | 2.34 |
| Acid functionality (theoretical) | 2.0 |
| Melting range (°C) | 65-67 |
| Tg (°C) | -43 |
| Tm (°C) | 65 |

b) Preparation of semi-crystalline polyglycidylester III

Preparation as in example 1b), using the semi-crystalline acid functional polyester 3 instead of 1.
The resulting white semi-crystalline product has the following properties:

| Molecular weight: | |
|---|---|
| $\overline{M}n$ | 1518 |
| $\overline{M}w$ | 2963 |
| Epoxy group content (found) (meq/g) | 1.96 |
| Epoxy group content (theoretical) (meq/g) | 2.09 |

(continued)

| Molecular weight: | |
|---|---|
| Tg (°) | -45 |
| Tm (°C) | 55 |

Example 4

a) Preparation of linear semi-crystalline acid functional polyester 4 using terephthalic acid as compound C

Example la was repeated using a mixture of 2.67 moles DDA and 1.33 moles terephthalic acid (TPA) instead of 4 moles DDA. TPA (1.33 moles) and HD (2.67 moles) were charged to the reactor (this time equipped with a pre-condensor to minimise glycol loss), heated to 230 °C and maintained at this temperature until the acid was below 0.1 meq/g.

The mixture was then cooled to 170 °C and DDA (2.67 moles) was added. The temperature was then raised to 210 °C and reaction was continued as described in example 1.

The product obtained was a white solid with the following properties:

| Molecular weight: | |
|---|---|
| $\overline{M}n$ | 1603 |
| $\overline{M}w$ | 3135 |
| Acid content (meq/g) | 2.60 |
| Acid functionality (theoretical) | 2.0 |
| Tg (°C) | -42 |
| Tm (°C) | 64 |

b) Preparation of semi-crystalline polyglycidylester IV

Preparation as example 1b using the semi-crystalline acid functional polyester 4 instead of 1.

The resulting white semi-crystalline product has the following properties:

| Molecular weight: | |
|---|---|
| $\overline{M}n$ | 1398 |
| $\overline{M}w$ | 3107 |
| Epoxy group content (found) (meq/g) | 2.00 |
| Epoxy group content (theoretical) (meq/g) | 2.28 |
| Tg (°C) | -32 |
| Tm (°C) | 37 |

Example 5

a) Preparation of a branched semi-crystalline acid functional polyester 5

Example 1a) was repeated using a mixture of 1.49 moles HD, 0.47 moles 1,1,1-tris-(hydroxymethyl)-propane(TMP) and 0.27 moles of pentaerythritol(PENTA) instead of 3 moles HD.

The product obtained was a white solid with the following properties:

| Molecular weight: | |
|---|---|
| $\overline{M}n$ | 2320 |
| $\overline{M}w$ | 55,000 |
| Acid content (meq/g) | 2.28 |
| Acid functionality (theoretical) | 3.33 |
| Melting range (°C) | 54-67 |
| Tm (°C) | 57 |

b) Preparation of semi-crystalline polyglycidylester V

Preparation as in example 1b, using the semi-crystalline acid functional polyester 5 instead of 1.
The resulting white semi-crystalline product has the following properties:

| Molecular weight: | |
| --- | --- |
| $\overline{M}n$ | 2280 |
| $\overline{M}w$ | 49,000 |
| Epoxy group content (found) (meq/g) | 2.00 |
| Epoxy group content (theoretical) (meq/g) | 2.05 |
| Melting range (°C) | 40-48 |
| Tm (°C) | 46 |
| Viscosity at 175 °C (mPa.s) | 350 |

Example 6

a) Preparation of a semi-crystalline acid functional branched polyester 6 using dimethylolpropionic acid as compound C

Example 1a) was repeated using a mixture of 1.2 moles HD, 0.45 moles TMP, 0.29 moles PENTA and 0.21 moles of dimethylolpropionic acid instead of 3 moles HD. The maximum temperature during the esterification reaction was 210 °C.
The obtained product was a white solid with the following properties:

| Molecular weight: | |
| --- | --- |
| $\overline{M}n$ | 2032 |
| $\overline{M}w$ | 59,700 |
| Acid content (meq/g) | 2.34 |
| Acid functionality (theoretical) | 3.67 |
| Melting range (°C) | 60-65 |
| Tm (°C) | 57 |
| Viscosity at 175 °C (mPa.s) | 800 |

b) Preparation of semi-crystalline polyglycidylester VI

Preparation as in example 1b), using the semi-crystalline acid functional polyester 6 instead of 1.
The resulting white semi-crystalline product has the following properties:

| Molecular weight: | |
| --- | --- |
| $\overline{M}n$ | 2356 |
| $\overline{M}w$ | 72,400 |
| Epoxy group content (found) (meq/g) | 2.11 |
| Epoxy group content (theoretical) (meq/g) | 2.17 |
| Tm (°C) | 45 |
| Viscosity at 175 °C (mPa.s) | 580 |

Examples 7 and 8 are thermosetting powder coating compositions according to the invention prepared with a polyglycidylester and an amorphous carboxyl group containing functional polyester.

Example 7

| Ingredients | Weight in g |
| --- | --- |
| Semi-crystalline Polyglycidylester V | 257.5 |

(continued)

| Ingredients | Weight in g |
|---|---|
| Carboxylic acid group containing, functional polyester[1] | 538.2 |
| Titanium dioxide | 159.1 |
| Modaflow III[2] | 9.6 |
| Benzoin | 6.3 |
| Actiron SNO 30[3] | 29.2 |

[1]Carboxylic functional polyester based on hydroxy pivalic acid (2 moles), dimethylolpropionic acid (2 moles), hydrogenated diphenylolpropane (6 moles), and hexahydrophthalic anhydride (9 moles) as disclosed in European patent application No. 93202081.1

[2]Modaflow III is an acrylic-based flow aid available from Monsanto Co.

[3]Actiron SNO 30 is a solid catalyst available from Protex.

The powder was prepared according to the following procedure.

The ingredients were mixed at room temperature, then melt-blended on a Buss single-screw extruder at 110 °C. The extrudate was chilled, flaked, ground in a micromill and classified through a 106 micrometre mesh.

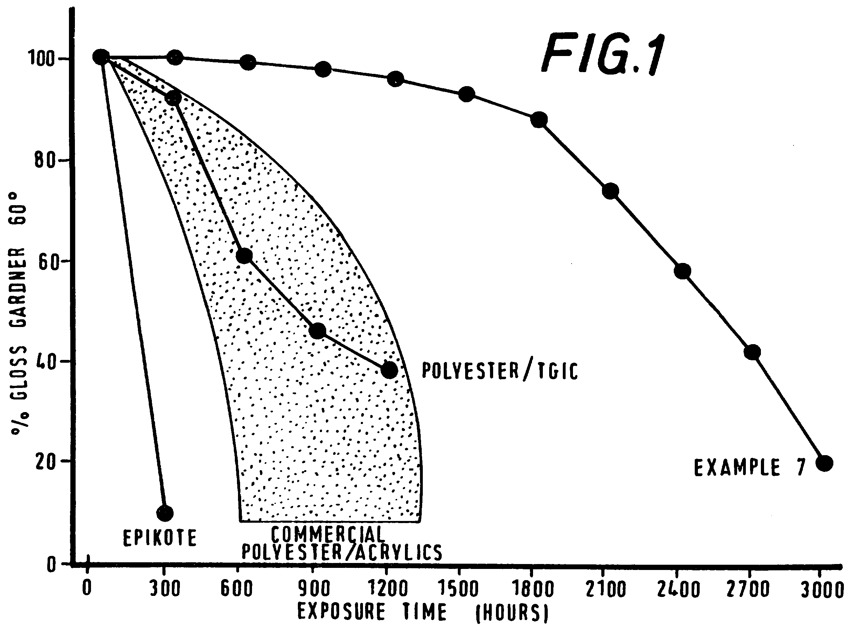

The powder was electrostatically sprayed onto chromate-pretreated, 2 mm thick aluminium panels. The coated panels were baked at 200 °C for 15 minutes. The resultant coatings (thickness 40-60 micrometres) were very smooth, hard, glossy and exhibited good mechanical and chemical properties as well as excellent weathering resistance, less than 10% reduction in gloss after exposure for 1200 hours in a Xenon type weather-O-meter running the SAE 1960 test method. A comparison with a powder coating composition based on a commercially available solid reaction product of 2,2-bis-(4-hydroxyphenyl)propane and epichlorohydrin (EPIKOTE 3003 ex. Shell Chemicals) and with a commercially available powder coating composition based on a polyester and TGIC (polyester/TGIC RAL9210 white) is given in figure 1 (% gloss gardner 60° versus exposure time in hours).

Example 8

| Ingredients | Weight in g |
|---|---|
| Semi-crystalline polyglycidylester V | 167.7 |
| Crylcoat E2988[1] | 651.8 |
| Titanium dioxide | 164.3 |
| Modaflow III[2] | 9.8 |
| Benzoin | 6.4- |

[1]Commercially available polyester ex UCB.

A powder coating was prepared according to the general instructions given in example 7. Coatings obtained were very smooth and glossy and exhibited good mechanical properties.

**Claims**

1. A semi-crystalline polyglycidylester obtainable by glycidation of the acid groups of a semi-crystalline acid functional polyester the latter being a reaction product of at least compound(s) A and compound(s) B, wherein compound A is a straight chain aliphatic alpha,omega-dicarboxylic acid having at least 6 carbon atoms, and wherein compound B is a straight chain aliphatic alpha,omega-primary diol having at least 4 carbon atoms, and optionally a compound C, having at least two functional groups reactive with hydroxyl and/or carboxyl groups and not falling within the definitions for compounds A and B, in an amount less than 25% by weight based on the total weight of the compounds A, B and C, said reaction product having an acid content of from $\geq 1.6$ meq/g to $\leq 4.0$ meq/g, a number average molecular weight of at least 500, a melting temperature (Tm) by Differential Scanning Calorimetry of $\geq 25$ °C, an average acid functionality of 2 or more and essentially no reactive groups other than carboxyl groups.

2. A polyglycidylester as claimed in claim 1 wherein compound A is adipic -, pimelic -, suberic -, azelaic -, sebacic - or 1,10-decanedicarboxylic acid.

3. A semi-crystalline polyglycidylester as claimed in claim 1 or 2 wherein compound B is 1,4-butanediol, 1,5-pentanediol, 1,6-hexanediol, 1,7-heptanediol, 1,8-octanediol, 1,9-nonane diol, 1,10-decanediol or 1,12-dodecanediol.

4. A semi-crystalline polyglycidylester as claimed in any one of the claims 1 to 3 wherein the semi-crystalline acid functional polyester is a reaction product of at least one compound A and one compound B and in addition at least one compound C having at least two functional groups reactive with hydroxyl- and/or carboxyl groups, not falling within the definitions given for compounds A and B and substantially free of any aromatic polycarboxylic acid.

5. A semi-crystalline polyglycidylester as claimed in claim 4 wherein compound C is an aliphatic or alicyclic diprimary amine, a cycloaliphatic polycarboxylic acid or its corresponding anhydride having at least two carboxyl groups per molecule, a polyol having at least three hydroxyl groups or a compound having simultaneously carboxyl- and hydroxyl groups and having a functionality of three or more.

6. A semi-crystalline polyglycidylester as claimed in claim 5 wherein compound C is hexanediamine, 1,1,1-tris(hydroxymethyl)propane, pentaerythritol or dimethylolpropionic acid.

7. A semi-crystalline polyglycidylester as claimed in any one of the claims 1 to 6 wherein the Tm of the semi-crystalline acid functional polyester is in the range of from 40 to 70 °C.

8. A semi-crystalline polyglycidylester as claimed in any one of the claims 1 to 7 wherein the acid functionality of the semi-crystalline acid functional polyester is from 2.0 to 3.5 carboxyl groups per molecule.

9. A semi-crystalline polyglycidyl ester as claimed in any one of the claims 1 to 8 wherein the acid content of the semi-crystalline acid functional polyester is of from 2.0 to 3.0 meq/g.

10. A process for the preparation of a semi-crystalline polyglycidylester as claimed in any one of the claims 1 to 9 by esterification of the compounds A, B and optionally C until essentially no free reactive groups other than carboxyl groups are present in the reaction mixture, followed by glycidation of the semi-crystalline acid functional polyester thus produced with epihalohydrin in the presence of a suitable base and catalyst.

11. A thermosetting powder coating composition comprising a semi-crystalline polyglycidylester as claimed in any one of the claims 1 to 9 and at least one suitable organic compound having groups reactive with epoxy groups and optionally a catalyst.

12. A thermosetting powder coating composition as claimed in claim 11 wherein the organic compound is a solid polyacid, an acid anhydride, an acid functional polyester, an acid functional polyacrylate, cyanuric acid or a solid basic curing agent.

13. A process for the preparation of a powder coating composition as claimed in claim 11 or 12 by blending all the constituents at ambient temperature followed by passing this premix through an extruder at a temperature sufficient to soften the semi-crystalline polyglycidylester and the organic compound but below the cure temperature, followed by cooling and grinding the extrudate.

14. Use of a powder coating composition as claimed claim 11 or 12 for coating a substrate therewith.

15. A substrate comprising a coating composition as claimed in claim 11 or 12 in the cured state.

**Patentansprüche**

1. Halbkristalliner Polyglycidylester, erhältlich durch Glycidierung der Säuregruppen eines halbkristallinen säurefunktionellen Polyesters, wobei der letztgenannte ein Reaktionsprodukt aus wenigstens einer Verbindung A und wenigstens einer Verbindung B, worin die Verbindung A eine geradkettige aliphatische α,omega-Dicarbonsäure mit wenigstens 6 Kohlenstoffatomen ist und worin die Verbindung B ein geradkettiges aliphatisches α,omega-primäres Diol mit wenigstens 4 Kohlenstoffatomen ist, und gegebenenfalls einer Verbindung C ist, die wenigstens 2, mit Hydroxyl- und/oder Carboxylgruppen reaktionsfähige funktionelle Gruppen aufweist und nicht unter die Definitionen für A und B fällt, in einer unter 25 Gew.-% liegenden Menge, bezogen auf das Gesamtgewicht der Verbindungen A, B und C, welches Reaktionsprodukt einen Säuregehalt von $\geq$ 1,6 mÄ/g bis $\leq$ 4,0 mÄ/g, ein zahlenmittleres Molekulargewicht von wenigstens 500, eine durch Differentielle Scanning-Kalorimetrie bestimmte Schmelztemperatur ($T_m$) von $\geq$ 25°C, eine mittlere Säurefunktionalität von 2 oder mehr und im wesentlichen keine anderen freien reaktionsfähigen Gruppen als Carboxylgruppen aufweist.

2. Polyester nach Anspruch 1, worin die Verbindung A Adipin-, Pimelin-, Suberin-, Azelain-, Sebacin- oder 1,10-Decandicarbonsäure ist.

3. Halbkristalliner Polyglycidylester nach Anspruch 1 oder 2, worin die Verbindung B 1,4-Butandiol, 1,5-Pentandiol, 1,6-Hexandiol, 1,7-Heptandiol, 1,8-Octandiol, 1,9-Nonandiol, 1,10-Decandiol oder 1,12-Dodecandiol ist.

4. Halbkristalliner Polyglycidylester nach einem der Ansprüche 1 bis 3, worin der halbkristalline säurefunktionelle Polyester ein Reaktionsprodukt von wenigstens einer Verbindung A und einer Verbindung B und zusätzlich wenigstens einer Verbindung C ist, die wenigstens zwei, mit Hydroxyl- und/oder Carboxylgruppen reaktionsfähige funktionelle Gruppen aufweist, nicht unter die für die Verbindungen A und B angegebenen Definitionen fällt und im wesentlichen frei von jeglicher aromatischer Polycarbonsäure ist.

5. Halbkristalliner Polyglycidylester nach Anspruch 4, worin die Verbindung C ein aliphatisches oder alicyclisches diprimäres Amin, eine cycloaliphatische Polycarbonsäure oder deren entsprechendes Anhydrid mit wenigstens 2 Carboxylgruppen je Molekül, ein Polyol mit wenigstens drei Hydroxylgruppen oder eine Verbindung ist, die gleichzeitig Carboxyl- und Hydroxylgruppen aufweist und eine Funktionalität von drei oder mehr zeigt.

6. Halbkristalliner Polyglycidylester nach Anspruch 5, worin die Verbindung C Hexandiamin, 1,1,1-Tris(hydroxymethyl)propan, Pentaerythrit oder Dimethylolpropionsäure ist.

7. Halbkristalliner Polyglycidylester nach einem der Ansprüche 1 bis 6, worin der Tm-Wert des halbkristallinen säurefunktionellen Polyesters im Bereich von 40 bis 70°C liegt.

8. Halbkristalliner Polyglycidylester nach einem der Ansprüche 1 bis 7, worin die Säurefunktionalität des halbkristallinen säurefunktionellen Polyesters von 2,0 bis 3,5 Carboxylgruppen je Molekül beträgt.

9. Halbkristalliner Polyglycidylester nach einem der Ansprüche 1 bis 8, worin der Säuregehalt des halbkristallinen säurefunktionellen Polyesters von 2,0 bis 3,0 Milliäquivalente/Gramm beträgt.

10. Verfahren zur Herstellung eines halbkristallinen Polyesters nach einem der Ansprüche 1 bis 9 durch Veresterung der Verbindungen A, B und gegebenenfalls C, bis im Reaktionsgemisch im wesentlichen keine anderen freien reaktionsfähigen Gruppen als Carboxylgruppen vorliegen, mit anschließender Glycidierung des so gebildeten halbkristallinen säurefunktionellen Polyesters mit Epihalogenhydrin in Anwesenheit einer geeigneten Base und eines Katalysators.

11. Hitzehärtende Pulverbeschichtungsmasse, umfassend einen halbkristallinen Polyglycidylester nach einem der Ansprüche 1 bis 9 und wenigstens eine geeignete organische Verbindung, die mit Epoxygruppen reaktionsfähige Gruppen aufweist, und gegebenenfalls einen Katalysator.

12. Hitzehärtende Pulverbeschichtungsmasse nach Anspruch 11, worin die organische Verbindung eine feste Polysäure, ein Säureanhydrid, ein säurefunktioneller Polyester, ein säurefunktionelles Polyacrylat, Cyanursäure oder ein festes basisches Härtungsmittel ist.

13. Verfahren zur Herstellung einer Pulverbeschichtungsmasse nach Anspruch 11 oder Anspruch 12 durch Vermischen sämtlicher Bestandteile bei Umgebungstemperatur und anschließendes Führen dieses Vorgemisches durch einen Extruder bei einer zum Erweichen des halbkristallinen Polyglycidylesters und der organischen Verbindung ausreichenden Temperatur, jedoch unter der Härtungstemperatur, gefolgt von einem Abkühlen und Mahlen des Extrudats.

14. Verwendung einer Pulverbeschichtungsmasse nach Anspruch 11 oder Anspruch 12 zum Überziehen eines Substrates.

15. Substrat, umfassend eine Beschichtungsmasse nach Anspruch 11 oder Anspruch 12 im gehärteten Zustand.

**Revendications**

1. Ester polyglycidylique semi-cristallin, que l'on peut obtenir par la glycidation des groupes acide d'un polyester acidofonctionnel semi-cristallin, ce dernier étant le produit de la réaction d'au moins un ou plusieurs composés (A) et un ou plusieurs composés (B), où le composé A est un acide alpha,oméga-dicarboxylique aliphatique à chaîne droite, possédant au moins 6 atomes de carbone, et où le composé B est un diol alpha,oméga-primaire aliphatique à chaîne droite, comportant au moins 4 atomes de carbone et éventuellement d'un composé C, possédant au moins deux radicaux fonctionnels réactifs avec les radicaux hydroxyle et/ou carboxyle et n'entrant pas dans les définitions données pour A et B, en une proportion inférieure à 25% en poids sur base du poids total des composants A, B et C, ledit produit de la réaction possédant une teneur en radicaux acide de ≥ 1,6 méq/g à ≤ 4,0 méq/g, un poids moléculaire moyen en nombre d'au moins 500, une température de fusion (Tf) selon la calorimétrie à exploration différentielle de ≥ 25°C, une fonctionnalité acide moyenne de 2 ou de plus de 2 et sensiblement dépourvu de radicaux réactifs autres que des groupes carboxyle.

2. Ester polyglycidylique suivant la revendication 1, caractérisé en ce que le composé A est l'acide adipique, pimellique, subérique, azélaïque, sébacique ou 1,10-décanedixarboxylique.

3. Ester polyglycidylique semi-cristallin suivant la revendication 1 ou 2, caractérisé en ce que le composé B est l'une des substances qui suivent : 1,4-butanediol, 1,5-pentanediol, 1,6-hexanediol, 1,7-heptanediol, 1,8-octanediol, 1,9-nonanediol, 1,10-décanediol et 1,12-dodécanediol.

4. Ester polyglycidylique semi-cristallin suivant l'une quelconque des revendications 1 à 3, caractérisé en ce que le polyester acidofonctionnel semi-cristallin est le produit de la réaction d'au moins un composé A et d'au moins un composé B et, en outre, d'au moins un composé C possédant au moins deux groupes fonctionnels réactifs avec les radicaux hydroxyle et/ou carboxyle, n'entrant pas dans le cadre des définitions données à propos des composés A et B et sensiblement dépourvus de tout acide polycarboxylique aromatique.

5. Ester polyglycidylique semi-cristallin suivant la revendication 4, caractérisé en ce que le composé C est un amine aliphatique ou alicyclique diprimaire, un acide polycarboxylique cycloaliphatique ou son anhydride correspondant, possédant au moins deux radicaux carboxyle par molécule, un polyol possédant au moins trois radicaux hydroxyle, ou un composé comportant simultanément des radicaux carboxyle et hydroxyle et possédant une fonctionnalité de trois et plus.

6. Ester polyglycidylique semi-cristallin suivant la revendication 5, caractérisé en ce que le composé C est l'hexanediamine, le 1,1,1-tris(hydroxyméthyl)propane, le pentaérythritol ou l'acide diméthylolpropionique.

7. Ester polyglycidylique semi-cristallin suivant l'une quelconque des revendications 1 à 6, caractérisé en ce que la Tf du polyester acidofonctionnel semi-cristallin varie de 40 à 70°C.

8. Ester polyglycidylique semi-cristallin suivant l'une quelconque des revendications 1 à 7, caractérisé en ce que la fonctionnalité acide du polyester acidofonctionnel semi-cristallin varie de 2,0 à 3,5 radicaux carboxyle par molécule.

9. Ester polyglycidylique semi-cristallin suivant l'une quelconque des revendications 1 à 8, caractérisé en ce que la teneur en acide du polyester acidofonctionnel semi-cristallin varie de 2,0 à 3,0 méq/g.

10. Procédé de préparation d'un ester polyglycidylique semi-cristallin suivant l'une quelconque des revendications 1 à 9 par l'estérification des composés A, B et, éventuellement, C jusqu'à ce qu'il ne subsiste sensiblement plus de radicaux réactifs libres autres que des groupes carboxyle dans le mélange réactionnel, suivie de la glycidation du polyester acidofonctionnel semi-cristallin ainsi produit avec une épihalohydrine en présence d'une base et d'un catalyseur convenables.

11. Composition de revêtement en poudre thermodurcissable qui comprend un ester polyglycidylique semi-cristallin suivant l'une quelconque des revendications 1 à 9 et au moins un composé organique approprié possédant des radicaux réactifs avec des groupes époxy et éventuellement un catalyseur.

12. Composition de revêtement en poudre thermodurcissable suivant la revendication 11, caractérisée en ce que le composé organique est un polyacide solide, un anhydride d'acide, un polyester acidofonctionnel, un polyacrylate acidofonctionnel, l'acide cyanurique ou un agent de durcissement basique solide.

**13.** Procédé de préparation d'une composition de revêtement en poudre suivant la revendication 11 ou 12 en mélangeant tous les constituants à la température ambiante, puis en faisant passer ce prémélange à travers une extrudeuse à une température suffisant à ramollir l'ester polyglydicylique semi-cristallin et le composé organique, mais inférieure à la température de durcissement, opération suivie du refroidissement et du broyage du produit extrudé.

**14.** Utilisation d'une composition de revêtement en poudre suivant la revendication 11 ou 12, pour le revêtement d'un subjectile à l'aide de cette composition.

**15.** Subjectile comprenant une composition de revêtement suivant la revendication 11 ou 12 à l'état durci.

# FIG.1

EPIKOTE

POLYESTER/TGIC

COMMERCIAL
POLYESTER/ACRYLICS

EXAMPLE 7

% GLOSS GARDNER 60°

EXPOSURE TIME (HOURS)

EP 0 751 970 B1